# EUROPEAN PATENT APPLICATION

(11) **EP 3 721 861 A1**
(43) Date of publication of application: **14.10.2020**
(21) Application number: 18885310.5
(22) Date of filing: 21.11.2018
(51) Int. Cl.: A61K 8/35, A61K 8/89, A61K 8/29, A61K 8/25, A61K 8/27, A61K 8/49, A61Q 17/04

(54) **ORGANIC/INORGANIC COMPOSITE, METHOD FOR PRODUCING SAME, AND ULTRAVIOLET BLOCKING AGENT USING SAME**

(30) Priority: 06.12.2017 KR 20170166762; 07.11.2018 KR 20180135542
(71) Applicant: SERA SOO CO., LTD, Chungcheongnam-do 31029 (KR)
(72) Inventor: RYU, Dong Seon, Seoul 06362 (KR)
(74) Representative: M. Zardi & Co S.A.
(86) International application number: PCT/KR2018/014304
(87) International publication number: WO 2019/112205

(57) **Abstract**

Are provided a novel organic-inorganic complex showing excellence in ultraviolet (UV) blocking effects, a manufacturing method thereof, and an UV blocking agents manufactured by using the same, and more specifically, there are provided a novel organic-inorganic complex capable of making up for the disadvantages of existing organic UV sunscreen materials and existing inorganic UV sunscreen materials, and increasing the advantages thereof, and a method of effectively manufacturing the organic-inorganic complex with a high yield of production, and an UV blocking agents manufactured by using the same.

## Description

### [Technical Field]

The present invention relates to a novel organic-inorganic complex composed of organic substance and inorganic substance and showing excellence in ultraviolet (UV) blocking effects, a manufacturing method thereof, and an UV blocking agents manufactured by using the same.

### [Background Art]

Generally, excessive exposure of ultraviolet (UV) radiation of the sunlight to skins is considered to be the leading cause of freckles or blemish generation by accelerating produce of reddening of skin, that is, erythema on skin or generate of melanin inside skin cells, or acknowledged to cause skin troubles by generating lipoperoxide from the reaction with sebum secreted by skin cells, and even in serious cases, to increase the risk of certain types of skin cancers. The UV radiation can be divided into UV-A (320∼400nm), UV-B (280∼320nm), and UV-C (200-280nm) in accordance with its wavelength, and toward going further shorter wavelength like UV-C, its energy is higher, but it is mostly absorbed by the atmosphere. Therefore, the UV radiation which directly affects on human bodies is known to as UV-A and UV-B.

Sunscreen compositions are commonly used to prevent sunburn and skin damages by such UV radiations, and they are typically classified as chemical sunscreens and physical sunscreens. Chemical sunscreen compositions, the fundamental mechanism of which is known to chemically absorb UV rays, are composed of organic sunscreen compounds such as, for example, cinnamic acid, salicylic acid, and benzophenone, etc. and physical sunscreen compositions, the fundamental mechanism of which is known as physical scattering and shielding of UV rays, are composed of inorganic sunscreen compounds such as, for example, titanium dioxide, and zinc oxide, etc.

The chemical sunscreen compositions are acknowledged to have advantages in good sunscreen effects but its applied wavelength ranges of UV rays are narrow and when it is employed for the components of cosmetic agents, it is not good because its usage feeling is sticky and greasy, and even since it might be absorbed by skins as molecular form, it has the disadvantage of causing skin irritation. In the case of the physical sunscreen compositions, skin irritation is relatively less than the chemical sunscreen compositions, but it has the disadvantages of heavy usage feeling and causing white case phenomenon on skins.

Various efforts and experiments about sunscreen compositions have been conducted in order to make up for these disadvantages of the chemical sunscreen compositions and the physical sunscreen compositions. First, as methods that most of UV sunscreen products employ at present, chemical sunscreen compositions and physical sunscreen compositions are mixed at appropriate rates, but disadvantages described above still fundamentally exist. Secondly, inorganic materials or polymer particle or organic sunscreen compositions are included on the surface or inside particles of inorganic sunscreen material, titanium dioxide, which supplements existing problems in the aspect of skin safety, but its manufacturing processes are very complex and the sunscreen block efficiency is not high enough. Thirdly, the disadvantages of present inorganic sunscreen compounds have been reduced and the efforts of pursuing the high efficiency of sunscreen block have been conducted by manufacturing the inorganic compounds such as titanium dioxide and zinc oxide as forms of ultrafine particles. However, white case phenomenon on skins cannot be completely removed, and it shows the disadvantage in the aspect of skin penetration as smaller its particle size becomes.

Recently, researches on organic-inorganic hybrid complex are continued to be as vigorous as ever in order to supplement the disadvantages of chemical organic sunscreen compositions and physical inorganic sunscreen compositions, but there still exist the disadvantages described above about chemical or physical sunscreen compositions, and are still remained the problems that the productivity, long-term stability, and the like are decreased.

The organic chemical compounds of formula 4 used in manufacturing organic-inorganic compounds hybrid complex shows excellent sunscreen block effect, but since it is strong acid material (pH: 0.9∼1.0), it should be always neutralized to basic substance in order to be used as cosmetic raw materials, and thus, a manufacturer 'L' company has conducted tests on many basic substances for neutralizing the materials. However, only triethanolamine is used in manufacturing cosmetics at present, but triethanolamine is known as material to cause skin allergies, and therefore, its usage for cosmetics manufacturing is being decreased.

### [PRIOR ART DOCUMENTS]

### [Patent Documents]

(Patent document 1) Korean Patent Registration No. 10-1575591 (Patent Registration Date 12. 08, 2015)
(Patent document 2) Korean Patent Application Publication No. 10-2014-0030395 (Patent Application Publication Date 03. 12, 2014)

### [Disclosure]

### [Technical Problem]

Therefore, it was required to solve the problems of conventional chemical and physical sunscreen compositions as above, and as the results of efforts to find new materials being excellent in sunscreen capability, there have been provided a novel organic-inorganic complex which is made by mixing organic compositions capable of effectively blocking ultraviolet (UV) radiations into inorganic compositions used as sunscreen materials, and a method of efficiently manufacturing this organic-inorganic complex. Therefore, in accordance with the present invention, there are provided an organic-inorganic complex having excellent UV sunscreen effects, a method of manufacturing thereof, and an UV blocking agent using the same.

### [Technical Solution]

Therefore, it is required to provide an organic-inorganic complex(composite) including chemical compounds indicated by chemical formula 1-1 or chemical formula 1-2 as shown in below.

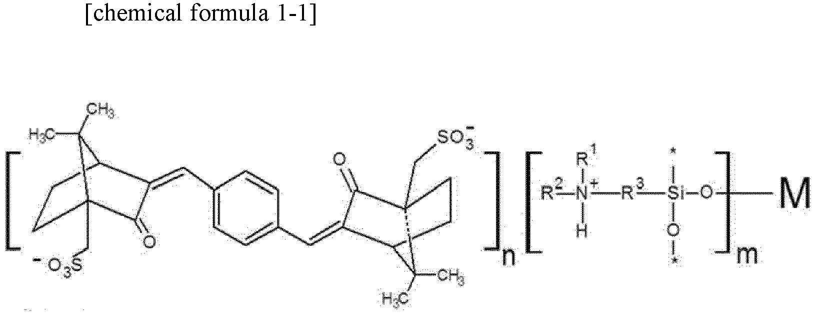

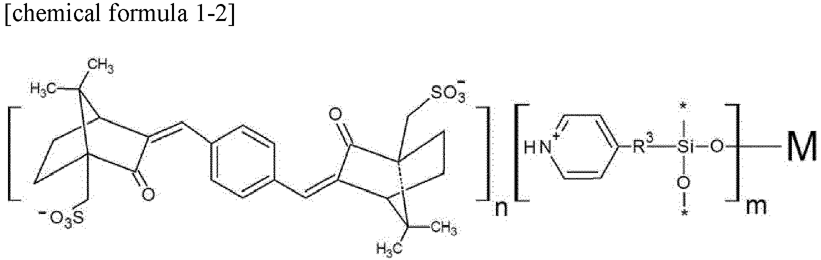

Herein, in the chemical formula 1-1 and the chemical formula 1-2, R¹ and R² may be independently hydrogen atom or alkyl group of C1∼C5 respectively, R³ may be alkylene group of C2∼C10, 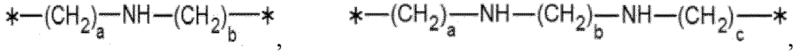 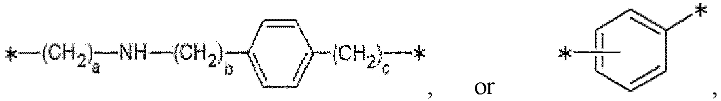 and a, b and c may be independently natural number of 1∼10 respectively, the coupler of Si indicated by * may bond with its neighboring atom of one kind or more selected from its neighboring O and C, and the coupler of O indicated by * may bond with its neighboring Si, and n and m may show the mole ratio of 0.5∼1.5: 1, and M may be inorganic oxide particle including one kind or more selected from TiO₂ particle, SiO₂ particle, and ZnO particle.

Further, another aspect of the present invention is to provide a method of manufacturing the organic-inorganic complex as above, and the method may include forming chemical compounds indicated by chemical formula 3-1 or chemical formula 3-2 below by reacting inorganic oxide particle and coupling agent indicated by chemical formula 2-1 or chemical formula 2-2 below; forming a dispersed solution by dispersing chemical compounds indicated by the chemical formula 3-1 or the chemical formula 3-2 into a solvent of pH 6.8∼7.2, and then, slowly adding drop by drop aqueous solution including chemical compounds indicated by chemical formula 4 below into the dispersed solution until reaching pH 6∼6.5, so as to form a reaction solution; and forming chemical compounds indicated by chemical formula 1-1 or chemical formula 1-2 below by stirring and reacting the reaction solution, and then, filtering, cleaning, and drying the chemical compounds.

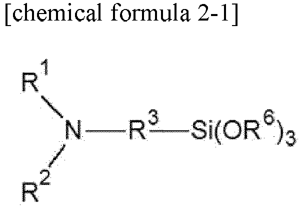

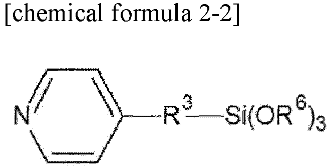

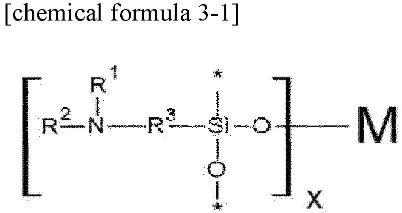

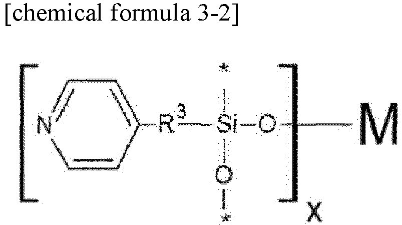

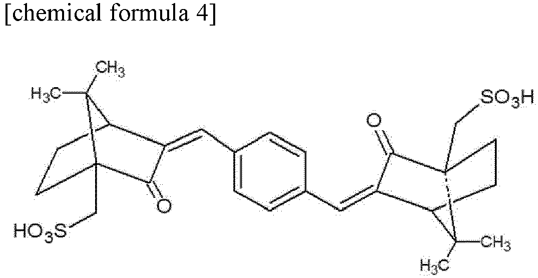

In the chemical formula 1-1 to the chemical formula 4, R¹ and R² as above may be independently hydrogen atom or alkyl group of C1∼C5 respectively, R³ may be alkyl group of C2∼C10, 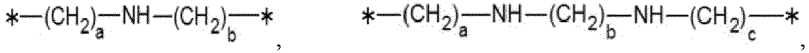 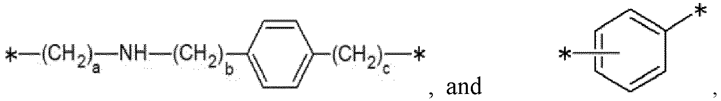 and R⁶ may be alkyl group of C1∼C5, or cycloalkyl group of C5∼C10, or penyl group, and a, b and c may be independently natural number of 1∼10 respectively, and the coupler of Si indicated by * may bond with its neighboring atom of one kind or more selected from its neighboring O and C, and the coupler of O indicated by * may bond with its neighboring Si, and the n and m may show the mole ratio of 0.5-1.5: 1, and m and x are the number of moles and rational number satisfying 0.3∼1.5mmol per 1g in mass of M, and M may be inorganic oxide particle including one kind or more selected from TiO₂ particle, SiO₂ particle, and ZnO particle.

Further, another aspect of the present invention is to provide an UV blocking agents which includes the organic-inorganic complex manufactured by the method as described above.

### [Advantageous Effects]

Therefore, the organic-inorganic complex manufactured in accordance with the present invention is little or very low in transdermal penetration as well as excellent in safety and in sun blocking capability throughout wide ranges of UV rays (UV-A, UV-B), and therefore, the UV blocking agents manufactured by using the same and having no side-effect against skin nor phototoxicity is provided. Further, by the method of manufacturing the organic-inorganic complex of the present invention, the organic-inorganic complex having the effects as above can be made in very high yield of production, and the UV blocking agents employed in cosmetics has advantages of no requiring neutralization process.

### [Best Mode]

### [Mode for Invention]

Hereinafter, the present invention will be described in more detail by the method of manufacturing organic-inorganic complex of the present invention.

The organic-inorganic complex of the present invention is manufactured by a first step of forming chemical compounds indicated by chemical formula 3-1 or chemical formula 3-2 below by reacting with inorganic oxide particle and the coupling agent indicated by chemical formula 2-1 or chemical formula 2-2 below, a second step of forming a dispersed solution by dispersing chemical compounds indicated by the chemical formula 3-1 or the chemical formula 3-2 into a solvent of pH 6.8∼7.2, and then, slowly adding drop by drop aqueous solution including chemical compounds indicated by chemical formula 4 below into the dispersed solution until reaching pH 6∼6.5, so as to form a reaction solution; and a third step of forming chemical compounds indicated by chemical formula 1-1 or chemical formula 1-2 below by stirring and reacting the reaction solution, and then, filtering, cleaning, and drying the chemical compounds.

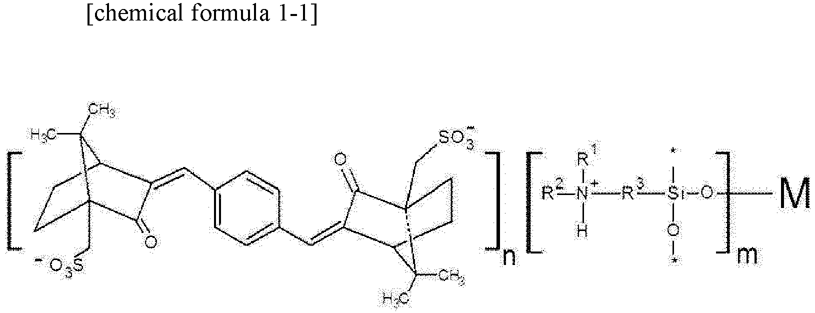

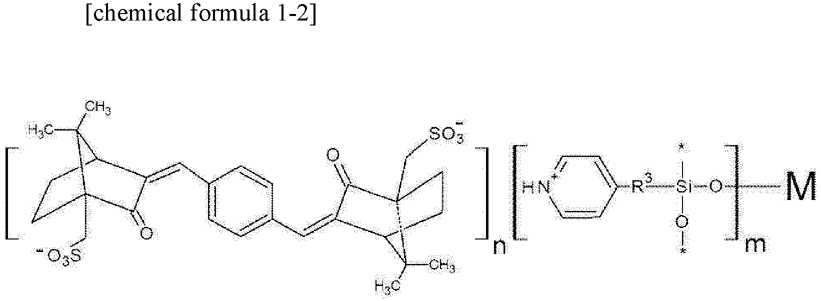

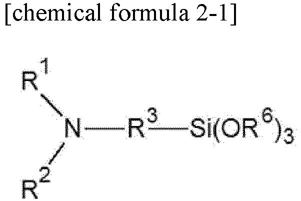

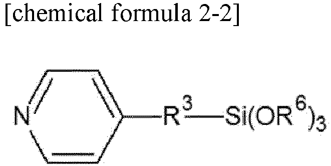

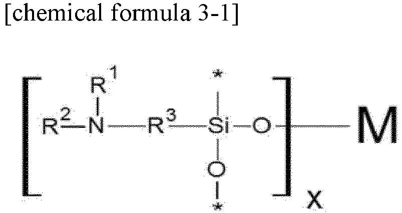

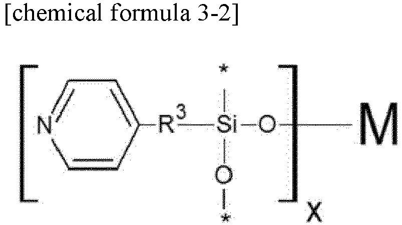

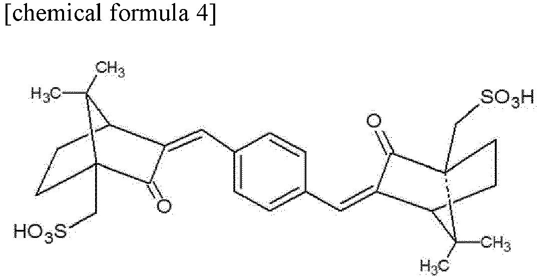

In the chemical formula 1-1 to the chemical formula 4, R¹ and R² are independently hydrogen atom or alkyl group of C1∼C5 respectively, and preferably, R¹ and R² are independently hydrogen atom or alkyl group of C1∼C3 respectively, and more preferably, R¹ and R² are independently hydrogen atom or methyl group.

Further, R³ is alkylene group of C2∼C10, 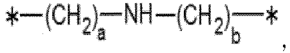 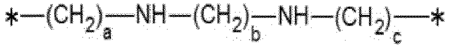 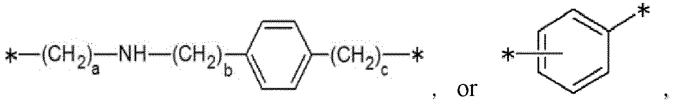 preferably, R³ is alkylene group of C2∼C5, 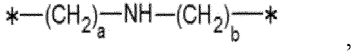 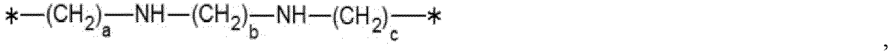 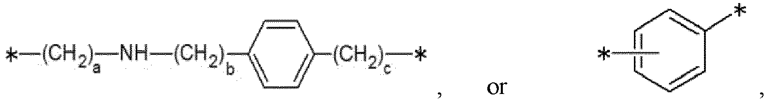 and more preferably, R³ is alkylene group of C3∼C4, 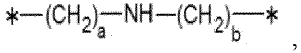 or 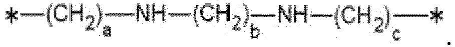 The a, b and c are natural number of 1∼10 respectively, and preferably, a, b and c are natural number of 1∼5 respectively

Further, the R⁶ of the chemical formula 2-1 or chemical formula 2-2 is alkyl group of C1∼C5, cycloalkyl group of C5∼C10, or penyl group, and preferably, R⁶ is alkyl group of C1∼C5, or cycloalkyl group of C5∼C10, and more preferably, R⁶ is linear alkyl group of C2∼C5.

Further, in the chemical formula 1-1 to chemical formula 4 as above, the coupler of Si indicated by * bonds with its neighboring O and/or C, the coupler of O indicated by * bonds with its neighboring Si, and as one example, it bonds (-(Si-O-Si-O)-) with the surface of inorganic oxide particle (M) in the form as shown in Fig. 1 below, and bonds with its neighboring functional group.

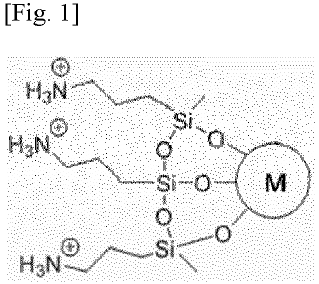

Further, in the chemical formula 1-1 to chemical formula 4 as above, the n and m show the mole ratio of 0.5∼1.5: 1, m and/or x is the number of mole and rational number satisfying 0.3∼1.5mmol per 1g in mass of M, and preferably, rational number satisfying 0.3∼1.0mmol per 1g in mass of M.

Further, in the chemical formula 1-1 to chemical formula 4 as above, the M is inorganic oxide particle including one kind or more selected from TiO₂ particle, SiO₂ particle, and ZnO particle.

In the manufacturing method according to the present invention, the inorganic oxide particle in the first step can be used from normal inorganic oxide particle used in the field of technology, and preferably, dry and modified-treated one can be used by removing moisture on the surface in order to improve the reactivity with coupling agent. In more specific, inorganic oxide particle is dried at the temperature of 100°C∼130°C for 1∼3 hours, and preferably, is dried at the temperature of 100°C∼120°C for 1.5∼2.5 hours. Then, the modified treatment can be performed by putting the dried inorganic oxide particle into anhydride toluene under argon gas and then, stirring for 30 minutes∼2 hours, and preferably for 50 minutes∼1.5 hours. Here, for the usage amount of the inorganic oxide particle, 1∼4g of inorganic oxide particle can be used per 20ml of anhydride toluene, preferably, 1.5∼3g, and more preferably, 1.5∼2.5g can be used. Here, if the usage amount of the inorganic oxide particle is less than 1g, it may cause the problem that the productivity in one process becomes too low, and if the usage amount of the inorganic oxide particle exceeds 4g, the stirring and mixing is not performed well, so that the reactivity with the chemical compound indicated by the chemical formula 2 is decreased.

The inorganic oxide particle corresponds to M of the compound indicated by chemical formula 1-1, chemical formula 1-2, chemical formula 3-1, and chemical formula 3-2, and normal inorganic oxide material used as UV blocking agents in the field of technology can be used, and preferably, may include one kind or two kinds or more selected from TiO₂ particle, SiO₂ particle, and ZnO particle. Further, the average particle diameter of the inorganic oxide particle is 10∼200nm, and more preferably, the average particle diameter of ZnO particle and TiO₂ particle is 10∼80nm, and the average particle diameter of SiO₂ particle is 80∼150nm. Here, if the average particle diameter of the inorganic oxide particle exceeds 200nm, it may cause the problem that UV blocking capability is decreased, and if the usage amount thereof is too much, it may cause white case phenomenon.

Further, for the process of manufacturing the chemical compound indicated by the chemical formula 3 of the first step, the chemical compound indicated by the chemical formula 2-1 or chemical formula 2-2 is input into anhydride toluene solution including the inroganic oxide particle under argon gas, and the mixed solution is refluxed for 10∼20 hours, and preferably for 12∼18 hours for reaction, and then, the reaction production is filtered, cleaned, and dried so that the chemical compound indicated by chemical formula 3 can be achieved.

The chemical compound indicated by chemical formula 2-1 or chemical formula 2-2 of the first step functions as coupling agent, and reacts with hydroxide group (-OH) which bonds with the surface of inorganic oxide particle, so as to bond with the surface of inorganic oxide particle, and produce the chemical compound indicated by chemical formula 3-1 or chemical formula 3-2 as reaction production.

The filtering of the first step can be performed by the normal method used in the field of technology, and the cleaning can be also performed by the normal method used in the field of technology, and preferably, the cleaning can be performed by repeatedly performing on the reaction production achieved by filtering with toluene. The drying can be also performed by the normal method used in the field of technology, and as one preferable method, the drying can be performed by vacuum-drying the cleaned reaction production for 4∼6 hours sufficiently and by removing toluene.

Then, the second step is the process of manufacturing the reaction production achieved by reacting the chemical compound indicated by chemical formula 3-1 or chemical formula 3-2, and the chemical compound indicated by chemical formula 4, and it is performed by, after inputting the chemical compound indicated by chemical formula 3 into a solvent of pH 6.8∼7.2, and preferably pH 6.9∼7.1, and stirring slowly for 30 minutes∼1.5 hours, producing dispersed solution by dispersing the chemical compound indicated by chemical formula 3 into the solvent. Here, for the solvent, distilled water can be used.

Further, the dispersed solution can be made by supplying the chemical compound per 50ml of the solvent, that is, by inputting 5∼17g, preferably 5∼14g, and more preferably 5.5∼12g of the chemical compound indicated by the chemical formula 3-1 or chemical formula 3-2 into the solvent, and if the usage amount of the chemical compound into the solvent is less than 5g, the productivity of the final reaction production becomes too low, but if the usage amount of the chemical compound into the solvent exceeds 17g, the stirring cannot be performed thoroughly and the chemical compound clumps on the bottom, so that the reaction speed with the chemical compound indicated by chemical formula 4 becomes decreased.

Further, while stirring the dispersed solution slowly, aqueous solution including chemical compound indicated by chemical formula 4 is added little by little into the dispersed solution. The adding is kept until the pH of mixing solution of the added solution and the dispersed solution reaches 6∼6.5, and preferably pH 6∼6.3. Here, when the adding is performed until pH exceeds 6.5, the reaction production which reacts less with the chemical compound indicated by chemical formula 4 is produced so that much amount of amine is remained, and UV blocking factor is decreased, but if the adding is performed until pH reaches less than pH6, it causes the problem that the acid component of the chemical compound indicated by chemical formula 4 cannot be neutralized completely.

Further, the aqueous solution including the chemical compound indicated by chemical formula 4 may include 30∼40 % by weight of the chemical compound indicated by chemical formula 4 and remaining amount of water, preferably, may include 30∼35 % by weight of the chemical compound indicated by chemical formula 4 and remaining amount of water, and more preferably, may include 32.6∼35.1 % by weight of the chemical compound indicated by chemical formula 4 and remaining amount of water. Here, the aqueous solution is announced to include 32.6∼35.1% by weight of the chemical compound indicated by chemical formula 4 by the Ministry of Food and Drug Safety.

Further, per 100 part by weight of the chemical compound indicated by chemical formula 3 in the step, the total added amount of the aqueous solution including the chemical compound indicated by chemical formula 4 may be 20∼90 part by weight, preferably, 25∼85 part by weight, and more preferably, 30∼78 part by weight.

Then, the third step is the process of stirring more for 4∼6 hours the added solution including the aqueous solution including the chemical compound indicated by chemical formula 4 into the dispersed solution of the second step, and producing reaction production made by reacting the chemical compound indicated by chemical formula 3 and the chemical compound indicated by chemical formula 4. The reaction production is the chemical compound indicated by the chemical formula 1-1 or chemical formula 1-2. By filtering, cleaning, and drying the reaction production made as above by the normal method used in the field of the technology, the organic-inorganic complex of the present invention can be manufactured in very high yield of production. In the organic-inorganic complex, 0.10∼0.35g of, and preferably 0.10∼0.30g of the chemical compound indicated by chemical formula 4 is bonded per 1g of the chemical compound indicated by chemical formula 3-1 or chemical formula 3-2.

The organic-inorganic complex of the present invention manufactured by the above method may include chemical compound indicated by the chemical formula 1-1 or chemical formula 1-2, and the organic-inorganic complex of the present invention as above can be used as the material for UV blocking agents.

Further, the organic-inorganic complex of the present invention shows high compatibility with existing and commercialized UV blocking agent components, and may be mixed and used with the UV blocking material including one kind or more selected from the group consisting of Glyceryl PABA, Drometrizole, Digalloyl Trioleate, 3-(4-Methylbenzylidene) Camphor, Menthyl Anthranilate, Benzophenone-3, Benzophenone-4, Benzophenone-8, Butyl Methoxydibenzoylmethane, Cinoxate, Octocrylene, Octyl Dimethyl-PABA, Octyl Methoxy Cinnamate, Octyl Salicylate, Octyltriazone, Para-aminobenzoic Acid, 2-Phenylbenzimidazole-5-sulfonic Acid, Homosalate, Drometrizole Trisiloxane, Disodium Phenyl Benzimidazole Tetrasulfonate, Bis-Ethylhexyloxyphenol Methoxyphenyl Triazine, and Isoamyl -p-methoxycinnamate.

The UV blocking agents manufactured by the method as above may be used as various forms of make-up materials such as kinds of makeup water, cream, powder, foundation, make-up base, shampoo, etc. for the components of functional cosmetic raw materials, as well as O/W (oil in water) sun cream, W/O (water in oil) sun cream, and specifically, can be employed as various phases such as emulsion, cream, paste, powder, solid, and the like, and ordinary methods of manufacturing normal cosmetic component materials can be used.

Now hereinafter, the present invention will be explained in more fully and detail in accordance with accompanying embodiments so that the present invention can be embodied and realized by those skilled in the art, and the exemplary embodiments of the present invention may be embodied in different forms and include equivalents that can realize the spirit of the present invention. It should be understood, however, that it is not intended to limit the present invention to the particular forms disclosed, but on the contrary, the present invention can be employed in more various shapes and the present invention is to cover all modifications, equivalents, and alternatives falling within the spirit and scope of the present invention.

### [EMBODIMENTS]

### Example 1: manufacturing of organic-inorganic complex (ZnO)

### (1) manufacturing of chemical compound indicated by chemical formula 3-1-1

After drying 2g of zinc oxide (ZnO) with average particle diameter of 15∼35nm in 110°C of a vacuum drier for 2 hours, by inputting into 200ml of anhydride toluene under argon gas, and for stirring for 1 hours, zinc oxide particle is prepared.

Then, by inputting 1ml of chemical compound indicated by chemical formula 2-1-1 as below into anhydride toluene solution including the zinc oxide particle, and refluxing for 15 hours under argon gas, reaction is performed. Then, by filtering and cleaning with toluene so as to remove non-reaction components, and vacuum-drying the reaction production for 5 hours so as to remove toluene, the chemical compound indicated by chemical formula 3-1-1 as below is manufactured.

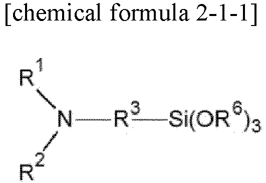

In the chemical formula 2-1-1, R³ is propyl group, R¹ and R² are hydrogen atoms, and R⁶ is methyl group.

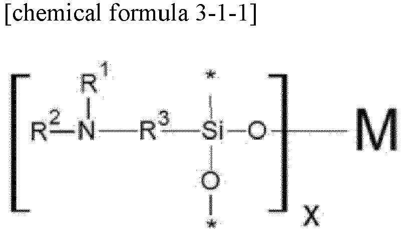

In chemical formula 3-1-1, R¹ and R² are hydrogen atoms, R³ is propyl group, M is zinc oxide particle, and x is rational number satisfying 0.8∼1mmol per 1g in mass of M.

### (2) manufacturing of chemical compound indicated by chemical formula 1-1-1

After putting 10g of chemical compound indicated by chemical formula 3-1-1 into 50ml of distilled water having pH 6.9∼7.0, by stirring for 1 hours, dispersed solution is manufactured.

Then, aqueous solution including the chemical compound indicated by chemical formula 4 with concentration of 33.5% by weight was added slowly drop by drop into the dispersed solution of the above until the pH of this mixing solution reaches 6.2∼6.3. The added amount of the aqueous solution including the chemical compound indicated by chemical formula 4 is 7.46g.

Then, by stirring the added solution (reaction solution) for 5 hours, and then, by filtering so as to remove non-reaction material, and by cleaning the filtered reaction production with alcohol, and then, by vacuum-drying at the temperature of 50°C, 17.45g of light yellow powder, which is organic-inorganic complex indicated by chemical formula 1-1-1 as below was obtained. The bonding rate of the chemical formula 3-1 and the chemical formula 4 in the manufactured organic-inorganic complex is about 0.25g of the compound indicated by chemical formula 4 per 1g of the compound indicated by chemical formula 3-1.

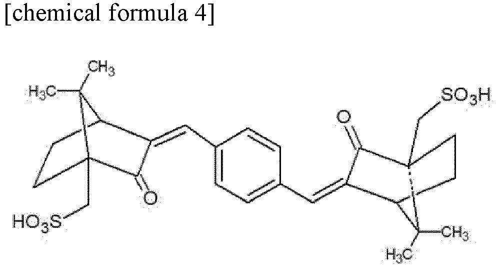

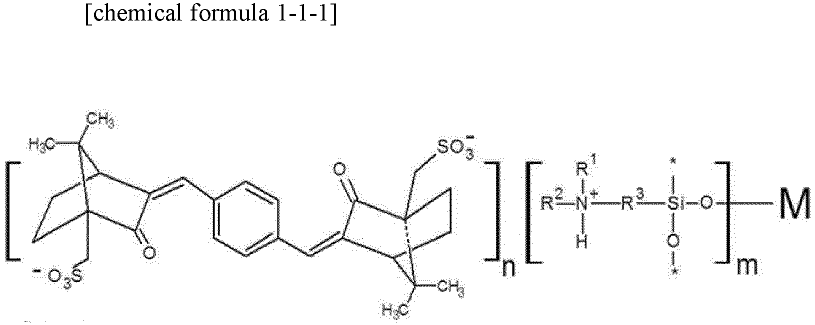

Herein, in the chemical formula 1-1-1, R¹ and R² are hydrogen atoms, R³ is propyl group, the coupler of Si indicated by * bonds with its neighboring O and/or C, the coupler of O indicated by * bonds with its neighboring Si, and the mole ratio of n and m is about 0.5:1, m and x are the number of moles and rational number satisfying 0.8∼1 mmol per 1g in mass of M, and M is ZnO particle.

### Example 2: manufacturing of organic-inorganic complex (TiO₂)

Organic-inorganic complex is manufactured by the same method as the Example 1, and by using TiO₂ particle with 15∼35nm of average particle diameter instead of ZnO particle, organic-inorganic complex including the chemical compound indicated by chemical formula 1-1-2 as below is manufactured. In the manufactured organic-inorganic complex, the bonding rate of the chemical formula 3-1-1 and chemical formula 4 is about 0.13g of the chemical compound indicated by chemical formula 4 per 1g of the chemical compound indicated by chemical formula 3-1-1.

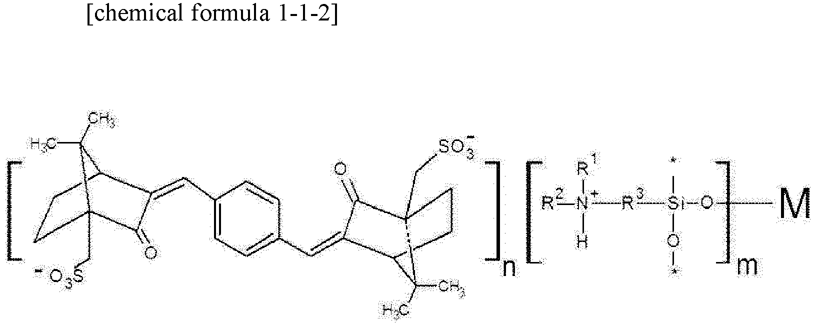

In the chemical formula 1-1-2, R¹ and R² are hydrogen atoms, R³ is propyl group, the coupler of Si indicated by * bonds with its neighboring O and/or C, the coupler of O indicated by * bonds with its neighboring Si, and the mole ratio of n and m is about 0.5:1, m is the number of mole and rational number satisfying 0.4∼0.5 mmol per 1g in mass of M, and the M is TiO₂ particle.

### Example 3: manufacturing of organic-inorganic complex (SiO₂)

Organic-inorganic complex is manufactured by the same method as the Example 1, and by using SiO₂ particle with 100∼150nm of average particle diameter instead of ZnO particle, organic-inorganic complex including chemical compound indicated by chemical formula 1-1-3 as below is manufactured. In the manufactured organic-inorganic complex, the bonding rate of chemical formula 3-1-1 and chemical formula 4 is about 0.25g of the chemical compound indicated by chemical formula 4 per 1g of the chemical compound indicated by chemical formula 3-1-1.

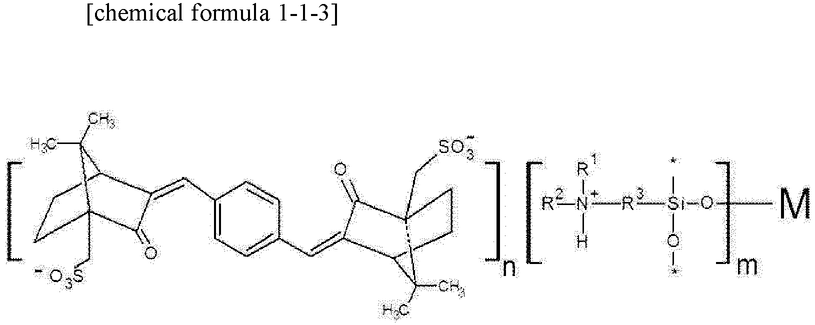

In the chemical formula 1-1-3, R¹ and R² are hydrogen atoms, R³ is propyl group, the coupler of Si indicated by * bonds with its neighboring O and/or C, the coupler of O indicated by * bonds with its neighboring Si, and the mole ratio of n and m is about 0.5:1, m is the number of mole and rational number satisfying 0.8∼1mmol per 1g in mass of M, and M is SiO₂ particle.

### Example 4

Organic-inorganic complex is manufactured by the same method as the Example 1, and by manufacturing chemical compound indicated by chemical formula 3-1-2 as below by using chemical compound indicated by chemical formula 2-1-2 as below, and by using this, organic-inorganic (ZnO) complex indicated by chemical formula 1-1-4 is manufactured in the embodiment 4.

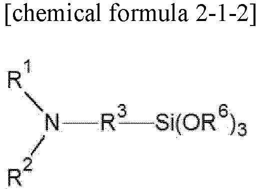

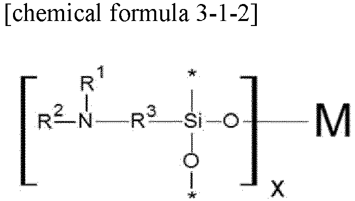

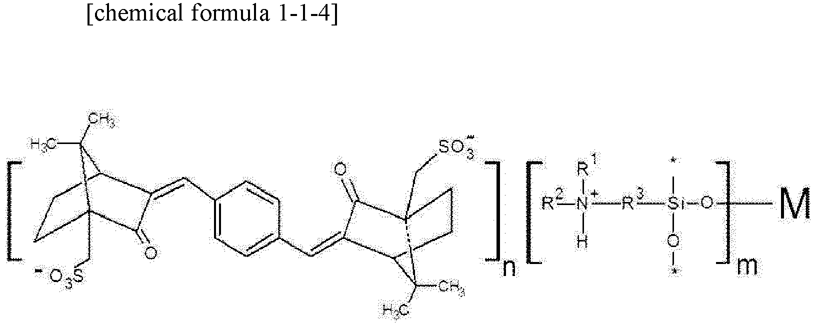

Herein, in the chemical formula 2-1-2, the chemical formula 3-1-2, and the chemical formula 1-1-4, R¹ and R² are hydrogen atoms, R³ is -(CH₂CH₂CH₂CH₂CH₂)-, R⁶ is methyl group (-CH₃), the coupler of Si indicated by * bonds with its neighboring O and/or C, the coupler of O indicated by * bonds with its neighboring Si, and the mole ratio of n and m is about 0.5:1, m is the number of mole and rational number satisfying 0.8∼1mmol per 1g in mass of M, and M is ZnO particle.

### Example 5

Organic-inorganic complex is manufactured by the same method as the Example 1, and by manufacturing chemical compound indicated by chemical formula 3-1-3 by using chemical compound indicated by chemical formula 2-1-3 as below, and by using this, organic-inorganic (ZnO) complex indicated by chemical formula 1-1-5 is manufactured in the embodiment 5.

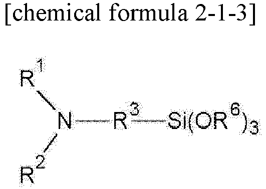

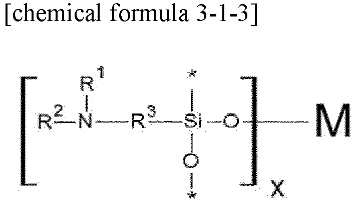

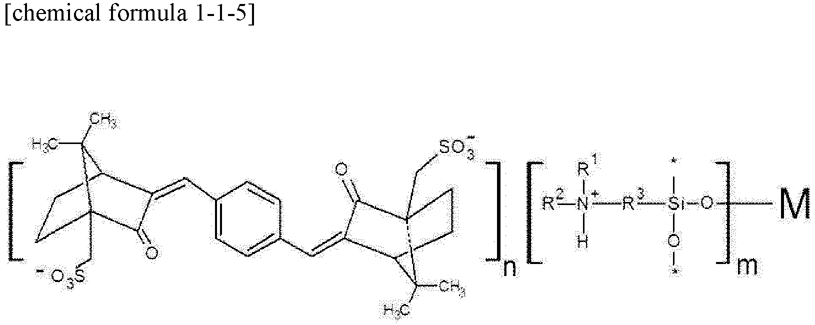

Herein, in the chemical formula 2-1-3, the chemical formula 3-1-3, and the chemical formula 1-1-5, R¹ and R² are hydrogen atoms, R³ is 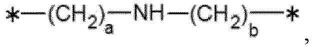 R⁶ is methyl group, the coupler of Si indicated by * bonds with its neighboring O and/or C, the coupler of O indicated by * bonds with its neighboring Si, and the mole ratio of n and m is about 0.5:1, m and x are the number of moles and rational number satisfying 0.3∼0.5mmol per 1g in mass of M, and M is ZnO particle.

### Example 6

Organic-inorganic complex is manufactured by the same method as the Example 1, and by manufacturing chemical compound indicated by chemical formula 3-1-4 by using chemical compound indicated by chemical formula 2-1-4 as below, and by using this, organic-inorganic (ZnO) complex indicated by chemical formula 1-1-6 is manufactured in the embodiment 6.

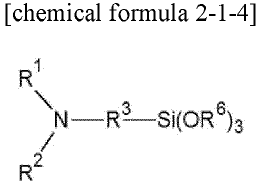

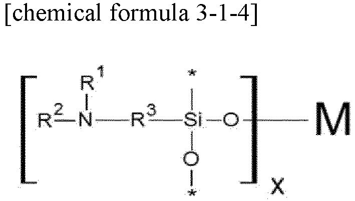

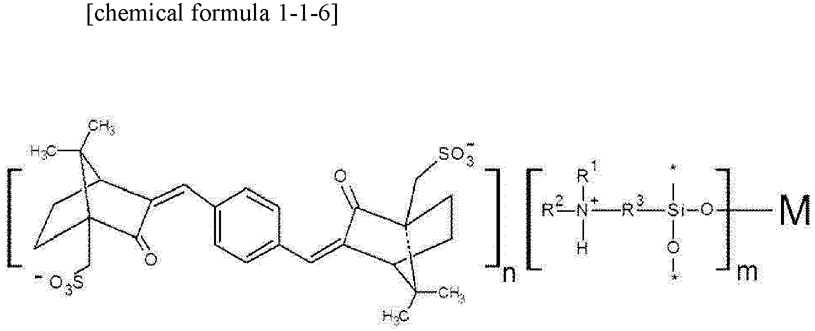

Herein, in the chemical formula 2-1-4, the chemical formula 3-1-4, and the chemical formula 1-1-6, R¹ and R² are hydrogen atoms, R³ is 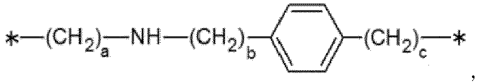 R⁶ is methyl group, the coupler of Si indicated by * bonds with its neighboring O and/or C, the coupler of O indicated by * bonds with its neighboring Si, and the mole ratio of n and m is about 0.5:1, m and x are the number of moles and rational number satisfying 0.3∼0.5mmol per 1g in mass of M, and M is ZnO particle.

### Example 7

Organic-inorganic complex is manufactured by the same method as the Example 1, and by manufacturing chemical compound indicated by chemical formula 3-2-1 by using chemical compound indicated by chemical formula 2-2-1 as below, and by using this, organic-inorganic (ZnO) complex indicated by chemical formula 1-2-1 is manufactured in the embodiment 7.

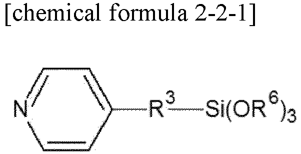

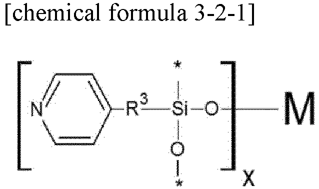

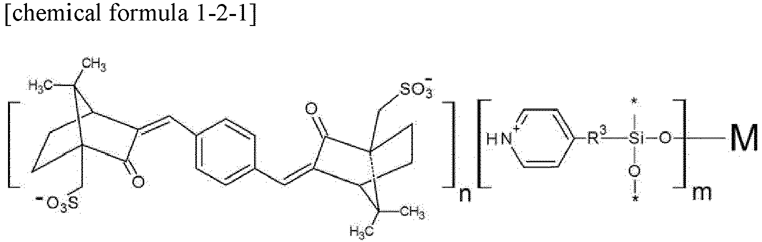

Herein, in the chemical formula 2-2-1, the chemical formula 3-2-1, and the chemical formula 1-2-1, R³ is propylene group, R⁶ is methyl group, the coupler of Si indicated by * bonds with its neighboring O and/or C, the coupler of O indicated by * bonds with its neighboring Si, and the mole ratio of n and m is about 0.5:1, m and x are the number of moles and rational number satisfying 0.3∼0.5mmol per 1g in mass of M, and M is ZnO particle.

### Example 8

Organic-inorganic complex is manufactured by the same method as the Example 1, and by manufacturing chemical compound indicated by chemical formula 3-2-2 by using chemical compound indicated by chemical formula 2-2-2 as below, and by using this, organic-inorganic (ZnO) complex indicated by chemical formula 1-2-2 is manufactured in the embodiment 8.

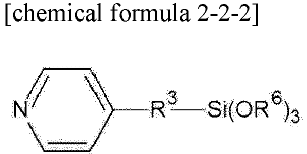

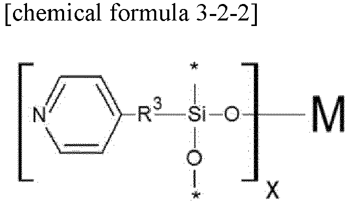

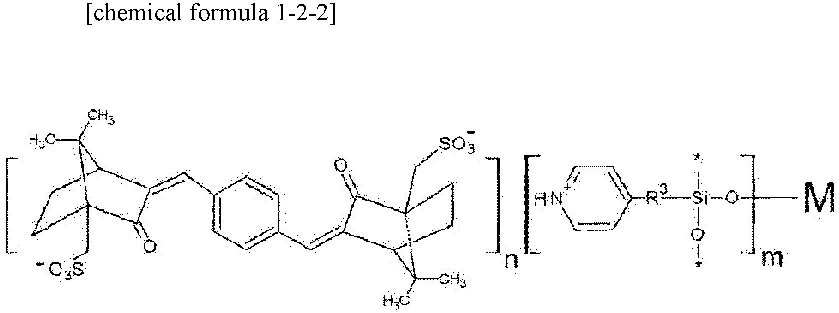

Herein, in the chemical formula 2-2-2, the chemical formula 3-2-2, and the chemical formula 1-2-2, R³ is 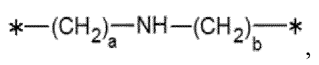 is methyl group, the coupler of Si indicated by * bonds with its neighboring O and/or C, the coupler of O indicated by * bonds with its neighboring Si, and the mole ratio of n and m is about 0.5:1, m and x are the number of moles and rational number satisfying 0.4∼0.6mmol per 1g in mass of M, and M is ZnO particle.

### Comparative Example 1

Organic-inorganic complex is manufactured by the same method as the Example 1, and in the comparative example 1, chemical compound indicated by chemical formula 3-1-5 is manufactured by using chemical compound indicated by chemical formula 2-1-5 as below, and by using this, organic-inorganic (ZnO) complex indicated by chemical formula 1-1-7 is manufactured.

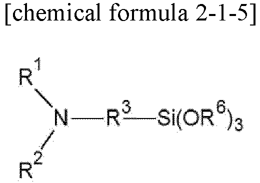

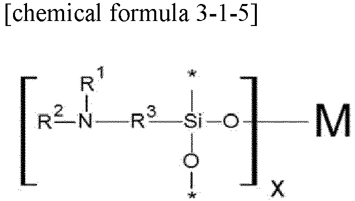

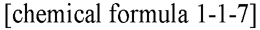

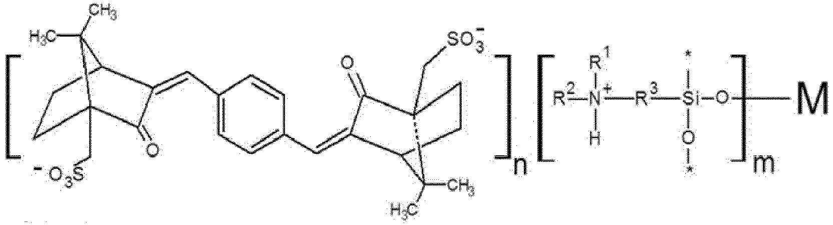

Herein, in the chemical formula 2-1-5, the chemical formula 3-1-5, and the chemical formula 1-1-7, R¹ and R² are hydrogen atoms, R³ is -(CH₂)-, R⁶ is methyl group, the coupler of Si indicated by * bonds with its neighboring O and/or C, the coupler of O indicated by * bonds with its neighboring Si, and the mole ratio of n and m is about 0.5:1, m and x are the number of moles and rational number satisfying 0.8∼1mmol per 1g in mass of M, and M is ZnO particle.

### Comparative Example 2

Organic-inorganic complex is manufactured by the same method as the Example 1, and in the comparative example 2, chemical compound indicated by chemical formula 3-1-6 is manufactured by using chemical compound indicated by chemical formula 2-1-6 as below, and by using this, organic-inorganic (ZnO) complex indicated by chemical formula 1-1-8 is manufactured.

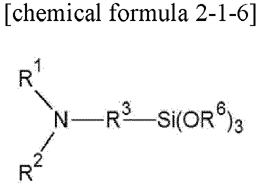

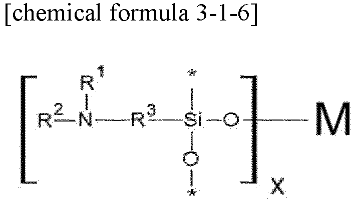

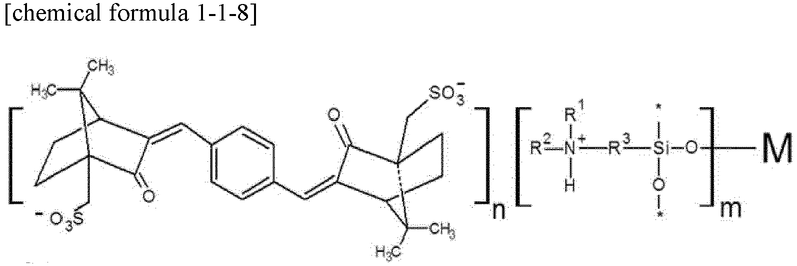

Herein, in the chemical formula 2-1-6, the chemical formula 3-1-6, and the chemical formula 1-1-8, R¹ and R² are hydrogen atoms, R³ is alkylene group having a carbon number of 12, R⁶ is methyl group, the coupler of Si indicated by * bonds with its neighboring O and/or C, the coupler of O indicated by * bonds with its neighboring Si, and the mole ratio of n and m is about 0.5:1, m and x are the number of moles and rational number satisfying 0.3∼0.5mmol per 1g in mass of M, and M is ZnO particle.

### Comparative Example 3

Organic-inorganic complex is manufactured by the same method as the Example 1, and in the comparative example 3, organic-inorganic complex is manufactured by using zinc oxide (ZnO) particle with average particle diameter of 220∼230nm instead of zinc oxide (ZnO) particle with average particle diameter of 15∼35nm.

### Manufacturing Example 1 and Comparative Manufacturing Example 1: Manufacturing of O/W (oil in water) sun-cream

By using the organic-inorganic complex manufactured in the Example 1 as UV blocking agents, O/W sun-cream is manufactured with the components and the amounts of Table 1 as shown below in the manufacturing Example 1. Then, measurement results of SPF and PA are shown in Table 1 as below.

In the comparative manufacturing example 1, sun-cream is manufactured by the same method as the manufacturing Example 1 except of mixing each component, 13, 14, and 15 without using the components of the Example 1.
A: The components of 1∼6 are heated and stirred at the temperature of 80°C.
B: The components of 7∼12 are heated and stirred at the temperature of 80°C.
C: while stirring, A mixing components are added into the B mixing components.
D: while maintaining the temperature below 60°C and stirring, organic-inorganic complex component of the Example 1 is added and manufactured. (manufacturing Example 1) / while maintaining the temperature below 60°C and stirring, components of 13, 14, and 15 are added and manufactured. (comparative manufacturing example 1)

**[Table 1]**

| number | Component name | | Manufacturing Example 1 (% by weight) | Comparative Manufacturing Example 1 (% by weight) |
|---|---|---|---|---|
| 1 | Propylene Glycol Dicaprylate | | 6.5 | 6.5 |
| 2 | Hydrogenated Polyisobutene | | 11.5 | 11.5 |
| 3 | dimethicone | | 1.5 | 1.5 |
| 4 | Behenyl alcohol | | 0.7 | 0.7 |
| 5 | Sorbitan Sesquistearate | | 0.5 | 0.5 |
| 6 | Glyceryl Stearate | | 0.3 | 0.3 |
| 7 | Sodium Acrylate/Sodium Acryloyldimethyl Taurate Copolymer | | 0.6 | 0.6 |
| 8 | Polysorbate 60 | | 1.0 | 1.0 |
| 9 | bentonite | | 0.1 | 0.1 |
| 10 | Microcrystalline Cellulose | | 2.5 | 2.5 |
| 11 | Butylene Glycol | | 6.0 | 6.0 |
| 12 | Purified water | | 48.8 | 48.8 |

| | Manufacturing Embodiment 1 | Comparative Manufacturing Example 1 | - | - |
|---|---|---|---|---|
| 13 | Organic-inorganic complex of embodiment 1 | Organic compound of chemical formula 4 | 20 | 4 |
| 14 | | ZnO | | 13.9 |
| 15 | | Triethanolamine | | 2.1 |
| SPF (UVB sun protection factor) | | | 27.4 | 21.4 |
| PA (protection grade of UVA) | | | 17.2 | 12.5 |

In order to measure UV rays blocking factors of the sun-cream manufactured by the manufacturing Example 1 and the comparative manufacturing example 1 respectively, in-vitro SPF & PA test (model name: SPF 290AS, manufacturer: Optometrics) was performed. The factors are repeatedly measured by three times, and the average number thereof was shown.

### Manufacturing Example 2 and Comparative Manufacturing Example 2: Manufacturing of O/W (oil in water) sun-cream

By using the organic-inorganic complex manufactured in the Example 2 as UV blocking agents, O/W sun-cream is manufactured with the components and the amounts of Table 2 as shown below in the manufacturing Example 2. Then, measurement results of SPF and PA are shown in Table 2 as below.

In the comparative manufacturing example 2, sun-cream is manufactured by the same method as the manufacturing Example 2 except of mixing each component, 13, 14, and 15 without using the components of the Example 2.
A: The components of 1∼6 are heated and stirred at the temperature of 80 °C .
B: The components of 7∼12 are heated and stirred at the temperature of 80°C.
C: while stirring, A mixing components are added into the B mixing components.
D: while maintaining the temperature below 60°C and stirring, organic-inorganic complex component of the Example 2 is added and manufactured. (manufacturing Example 2) / while maintaining the temperature below 60°C and stirring, components of 13, 14, and 15 are added and manufactured. (comparative manufacturing example 2)

**[Table 2]**

| number | Component name | | Manufacturing Example 2 (% by weight) | Comparative Manufacturing Example 2 (% by weight) |
|---|---|---|---|---|
| 1 | Propylene Glycol Dicaprylate | | 6.5 | 6.5 |
| 2 | Hydrogenated Polyisobutene | | 11.5 | 11.5 |
| 3 | dimethicone | | 1.5 | 1.5 |
| 4 | Behenyl alcohol | | 0.7 | 0.7 |
| 5 | Sorbitan Sesquistearate | | 0.5 | 0.5 |
| 6 | Glyceryl Stearate | | 0.3 | 0.3 |
| 7 | Sodium Acrylate/ Sodium Acryloyldimethyl Taurate Copolymer | | 0.6 | 0.6 |
| 8 | Polysorbate 60 | | 1.0 | 1.0 |
| 9 | bentonite | | 0.1 | 0.1 |
| 10 | Microcrystalline Cellulose | | 2.5 | 2.5 |
| 11 | Butylene Glycol | | 6.0 | 6.0 |
| 12 | Purified water | | 48.8 | 48.8 |

| | Manufacturing Embodiment 2 | Comparative Manufacturing Example 2 | - | - |
|---|---|---|---|---|
| 13 | Organic-inorganic complex of embodiment 2 | Organic compound of chemical formula 4 | 20 | 2 |
| 14 | | TiO₂ | | 17 |
| 15 | | Triethanolamine | | 1 |
| SPF (UVB sun protection factor) | | | 31.9 | 23.4 |
| PA (protection grade of UVA) | | | 16.1 | 11.2 |

In order to measure UV rays blocking factors of the sun-cream manufactured by the manufacturing Example 2 and the comparative manufacturing example 2 respectively, in-vitro SPF & PA test (model name: SPF 290AS, manufacturer: Optometrics) was performed. The factors are repeatedly measured by three times, and the average number thereof was shown.

### Manufacturing Example 3 and Comparative Manufacturing Example 3: Manufacturing of O/W (oil in water) sun-cream

By using the organic-inorganic complex manufactured in the Example 3 as UV blocking agents, O/W sun-cream is manufactured with the components and the amounts of Table 3 as shown below in the manufacturing Example 3. Then, measurement results of SPF and PA are shown in Table 3 as below.

In the comparative manufacturing example 3, sun-cream is manufactured by the same method as the manufacturing Example 3 except of mixing each component, 13, 14, and 15 without using the components of the Example 3.
A: The components of 1∼6 are heated and stirred at the temperature of 80 °C .
B: The components of 7∼12 are heated and stirred at the temperature of 80°C .
C: while stirring, A mixing components are added into the B mixing components.
D: while maintaining the temperature below 60°C and stirring, organic-inorganic complex component of the Example 3 is added and manufactured. (manufacturing Example 3) / while maintaining the temperature below 60°C and stirring, components of 13, 14, and 15 are added and manufactured. (comparative manufacturing example 3)

**[Table 3]**

| number | Component name | | Manufacturing Example 3 (% by weight) | Comparative Manufacturing Example 3 (% by weight) |
|---|---|---|---|---|
| 1 | Propylene Glycol Dicaprylate | | 6.5 | 6.5 |
| 2 | Hydrogenated Polyisobutene | | 11.5 | 11.5 |
| 3 | dimethicone | | 1.5 | 1.5 |
| 4 | Behenyl alcohol | | 0.7 | 0.7 |
| 5 | Sorbitan Sesquistearate | | 0.5 | 0.5 |
| 6 | Glyceryl Stearate | | 0.3 | 0.3 |
| 7 | Sodium Acrylate/Sodium Acryloyldimethyl Taurate Copolymer | | 0.6 | 0.6 |
| 8 | Polysorbate 60 | | 1.0 | 1.0 |
| 9 | bentonite | | 0.1 | 0.1 |
| 10 | Microcrystalline Cellulose | | 2.5 | 2.5 |
| 11 | Butylene Glycol | | 6.0 | 6.0 |
| 12 | Purified water | | 48.8 | 48.8 |

| | Manufacturing Embodiment 3 | Comparative Manufacturing Example 3 | - | - |
|---|---|---|---|---|
| 13 | Organic-inorganic complex of embodiment 3 | Organic compound of chemical formula 4 | 20 | 4 |
| 14 | | SiOz | | 13.9 |
| 15 | | Triethanolamine | | 2.1 |
| SPF (UVB sun protection factor) | | | 22.4 | 16.9 |
| PA (protection grade of UVA) | | | 14.2 | 9.7 |

In order to measure UV rays blocking factors of the sun-cream manufactured by the manufacturing Example 3 and the comparative manufacturing example 3 respectively, in-vitro SPF & PA test (model name: SPF 290AS, manufacturer: Optometrics) was performed. The factors are repeatedly measured by three times, and the average number thereof was shown.

### Manufacturing Example 4∼8 and Comparative Manufacturing Examples 4∼6: Manufacturing of O/W (oil in water) sun-cream

O/W sun-creams are manufactured respectively by the same method as in the manufacturing Example 1, but manufactured respectively by varying organic-inorganic complex as shown in Table 4 as below, and the SPF and PA measurement results thereof are shown in Table 4 as below.

**[Table 4]**

| classification | organic-inorganic complex | SPF (UVB sun protection factor) | PA (protection grade of UVA) |
|---|---|---|---|
| Manufacturing Example 1 | Example 1 | 27.4 | 17.2 |
| Manufacturing Example 4 | Example 4 | 28.2 | 16.3 |
| Manufacturing Example 5 | Example 5 | 31.5 | 15.1 |
| Manufacturing Example 6 | Example 6 | 26.9 | 16.7 |
| Manufacturing Example 7 | Example 7 | 23.5 | 14.0 |
| Manufacturing | Example 8 | 24.8 | 14.5 |
| Example 8 | | | |
| Comparative Manufacturing Example 4 | Comparative Example 1 | 19.5 | 12.1 |
| Comparative Manufacturing Example 5 | Comparative Example 2 | 28.5 | 15.8 |
| Comparative Manufacturing Example 6 | Comparative Example 3 | 18.2 | 10.6 |

In view of the experiment results as shown in Table 4 as above, and in the case of manufacturing Example 1 to 8, the experiment results show that SPF is 20 or higher and preferably 23 or higher, and PA is 13 or higher and preferably 14.0 or higher so that the sun blocking factors of UVB and UVA are excellent. On the contrary, in the sun-cream of the comparative manufacturing example 4 made by using the organic-inorganic complex of comparative example 1, SPF and PA are rapidly decreased in comparison with in the manufacturing Example 1. In the sun-cream of the comparative manufacturing example 5 made by using the organic-inorganic complex of comparative example 2, SPF is excellent, but PA is rapidly decreased in comparison with in the manufacturing Example 1 and the manufacturing Example 4. In the case of the sun-cream manufactured in the comparative manufacturing example 6 by using the organic-inorganic complex made by using ZnO particle with particle diameter higher than 200nm, it causes problems in that ordinary sun blocking capabilities are decreased in comparison with in the manufacturing Example 1 as well as white case phenomenon is occurred.

As described above, the organic-inorganic complex of the present invention throughout the Examples and the manufacturing Examples as above shows excellence in stability, and the sun blocking capability is also excellent through wide ranges of UV rays (UV-A and UV-B). Further, the organic-inorganic complex as in the present invention shows much higher values in SPF and PA rather than using organic UV sun-screen agent or inorganic UV sun-screen agent separately.

## Claims

1. An organic-inorganic complex including chemical compounds indicated by chemical formula 1-1 or chemical formula 1-2 below, 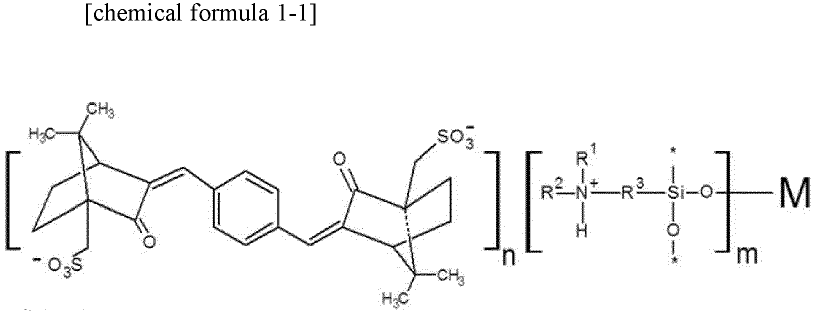 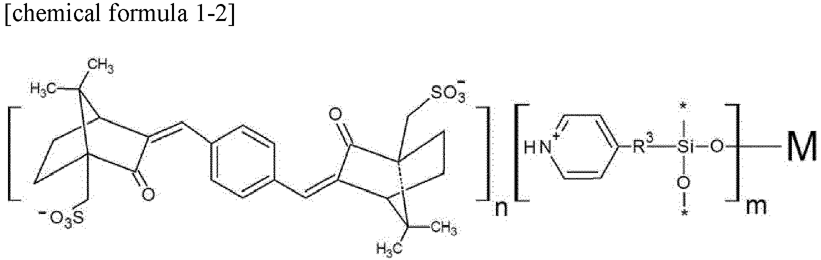 in which in the chemical formula 1-1 and the chemical formula 1-2, R¹ and R² are independently hydrogen atom or alkyl group of C1∼C5 respectively, R³ is alkylene group of C2∼C10, 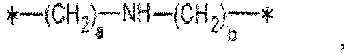 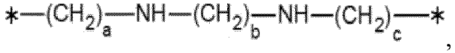 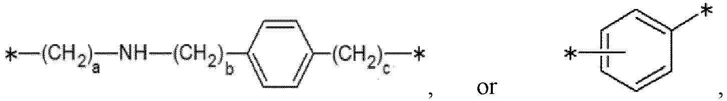 a, b and c are independently natural number of 1∼10 respectively, the coupler of Si indicated by * bonds with its neighboring atom of one kind or more selected from its neighboring O and C, and the coupler of O indicated by * bonds with its neighboring Si, and n and m show the mole ratio of 0.5∼1.5: 1, and M is inorganic oxide particle including one kind or more selected from TiO₂ particle, SiO₂ particle, and ZnO particle.

2. The organic-inorganic complex as claimed in claim 1,
wherein R¹ and R² in the chemical formula 1-1 and the chemical formula 1-2 are independently hydrogen atom or methyl group respectively, R³ is alkyl group of C2∼C5, and a, b, and c are natural number of 1∼5 respectively.

3. A method of manufacturing an organic-inorganic complex comprising:
a first step of forming chemical compounds indicated by chemical formula 3-1 or chemical formula 3-2 below by reacting inorganic oxide particle and coupling agent indicated by chemical formula 2-1 or chemical formula 2-2 below;
a second step of forming a dispersed solution by dispersing chemical compounds indicated by the chemical formula 3-1 or the chemical formula 3-2 into a solvent of pH 6.8∼7.2, and then, slowly adding drop by drop aqueous solution including chemical compounds indicated by chemical formula 4 below into the dispersed solution until reaching pH 6∼6.5, so as to form a reaction solution; and
a third step of forming chemical compounds indicated by chemical formula 1-1 or chemical formula 1-2 below by stirring and reacting the reaction solution, and then, filtering, cleaning, and drying the chemical compounds, in which 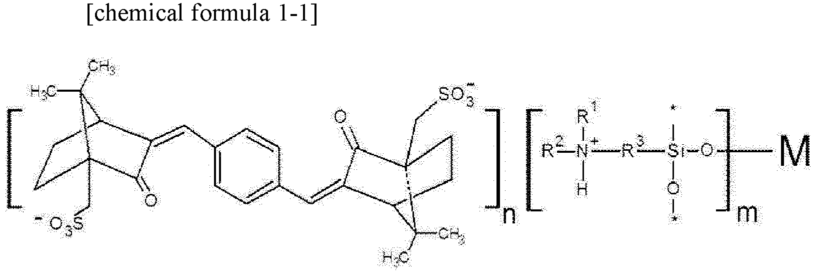 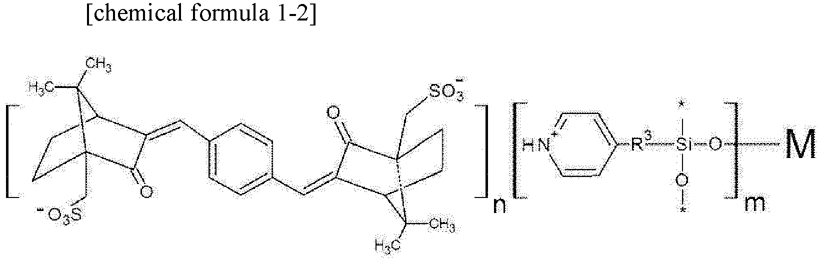 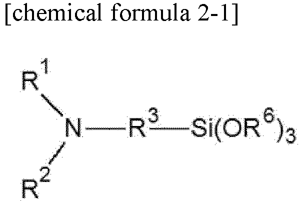 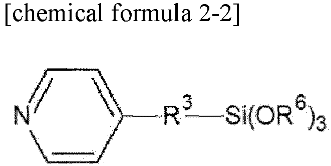 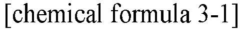 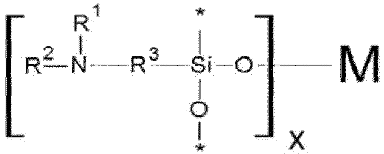 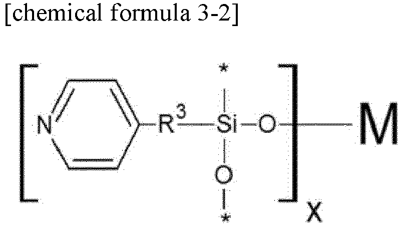 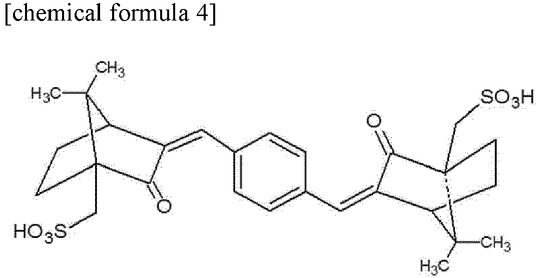
and in the chemical formula 1-1 to the chemical formula 4, R¹ and R² are independently hydrogen atom or alkyl group of C1∼C5 respectively, R³ is alkyl group of C2∼C10, 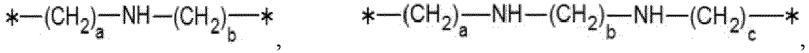 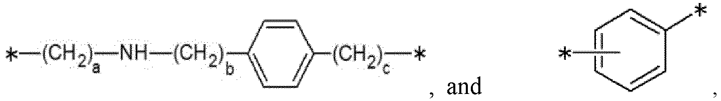 and R⁶ is alkyl group of C1∼C5, or cycloalkyl group of C5∼C10, or penyl group, and a, b and c are independently natural number of 1∼10 respectively, and the coupler of Si indicated by * bonds with its neighboring atom of one kind or more selected from its neighboring O and C, and the coupler of O indicated by * bonds with its neighboring Si, and n and m show the mole ratio of 0.5-1.5: 1, and m and x are the number of moles and rational number satisfying 0.3∼1.5mmol per 1g in mass of M, and M is inorganic oxide particle including one kind or more selected from TiO₂ particle, SiO₂ particle, and ZnO particle.

4. The method of manufacturing an organic-inorganic complex as claimed in claim 3,
wherein the average particle diameter of the inorganic oxide particle is 10∼200nm.

5. The method of manufacturing an organic-inorganic complex as claimed in claim 3,
wherein the first step is to provide chemical compounds indicated by the chemical formula 3 by inputting the chemical compounds indicated by the chemical formula 2 into anhydrous toluene solution including modified inorganic oxide particle under argon gas, and then, performing reaction by refluxing for 10∼20 hours, and filtering, cleaning, and drying the reaction product.

6. The method of manufacturing an organic-inorganic complex as claimed in claim 3,
wherein the dispersed solution in the second step is made by inputting 5∼17g of chemical compounds indicated by the chemical formula 3-1 or the chemical formula 3-2 into solvent per 50ml, and then stirring it.

7. The method of manufacturing an organic-inorganic complex as claimed in claim 3,
wherein the aqueous solution including chemical compounds indicated by chemical formula 4 in the second step comprises 30∼40% by weight of the chemical compounds indicated by chemical formula 4 and remaining amount of water.

8. The method of manufacturing an organic-inorganic complex as claimed in claim 3,
wherein 20∼90 part by weight of aqueous solution including chemical compounds indicated by chemical formula 4 based on 100 part by weight of the chemical compounds indicated by chemical formula 3-1 or chemical formula 3-2 of the second step is used.

9. An UV blocking agents comprising the organic-inorganic complex of claim 1 or claim 2.

10. The UV blocking agents as claimed in claim 9,
wherein the UV blocking agents further comprise UV blocking material composed of one kind or more selected from Glyceryl PABA, Drometrizole, Digalloyl Trioleate, 3-(4-Methylbenzylidene) Camphor, Menthyl Anthranilate, Benzophenone-3, Benzophenone-4, Benzophenone-8, Butyl Methoxydibenzoylmethane, Cinoxate, Octocrylene, Octyl Dimethyl-PABA, Octyl Methoxy Cinnamate, Octyl Salicylate, Octyltriazone, Para-aminobenzoic Acid, 2-Phenylbenzimidazole-5-sulfonic Acid, Homosalate, Drometrizole Trisiloxane, Disodium Phenyl Benzimidazole Tetrasulfonate, Bis-Ethylhexyloxyphenol Methoxyphenyl Triazine, and Isoamyl -p-methoxycinnamate.
